# EUROPEAN PATENT SPECIFICATION

(11) **EP 2 005 163 B1**
(45) Date of publication and mention of the grant of the patent: **28.09.2016**
(21) Application number: 07732202.2
(22) Date of filing: 29.03.2007
(51) Int. Cl.: G01N 33/50

(54) **METHOD FOR CELL BASED ASSAYS**
METHODE FÜR TESTVERFAHREN AUF ZELLBASIS
PROCÉDÉ RELATIF À DES ESSAIS À BASE DE CELLULES

(30) Priority: 31.03.2006 GB 0606430
(43) Date of publication of application: 24.12.2008
(73) Proprietor: GE Healthcare Limited, Little Chalfont, Buckinghamshire HP7 9NA (GB)
(72) Inventor: THOMAS, Nicholas, Cardiff CF14 7YT (GB); KENRICK, Michael, Kenneth, Cardiff CF14 7YT (GB); MURATHODZIC, Sharon, Joanne, Bridgend CF31 5BL (GB)
(74) Representative: Aldenbäck, Ulla Christina
(86) International application number: PCT/GB2007/001148
(87) International publication number: WO 2007/113496

(56) References cited:
- WO-A-99/21962
- GB-A- 2 059 991
- US-A1- 2002 094 543
- FURSOV NATALIE ET AL: "IMPROVING CONSISTENCY OF CELL-BASED ASSAYS BY USING DIVISION-ARRESTED CELLS" ASSAY AND DRUG DEVELOPMENT TECHNOLOGIES, MARY ANN LIEBERT, NEW YORK, NY, US, vol. 3, no. 1, February 2005 (2005-02), pages 7-15, XP008069316 ISSN: 1540-658X
- YANAI N ET AL: "CRYOPRESERVATION OF ANIMAL CELLS GROWING ON MICROCARRIERS" BIOLOGICAL ABSTRACTS. - MICROFILMS, BIOSIS. PHILADELPHIA, PA, US, vol. 88, no. 9, 1989, page AB240, XP002060204 ISSN: 0192-6985
- DIGAN MARY ELLEN ET AL: "EVALUATION OF DIVISION-ARRESTED CELLS FOR CELL-BASED HIGH-THROUGHPUT SCREENING AND PROFILING" JOURNAL OF BIOMOLECULAR SCREENING, LARCHMONT, NY, US, vol. 10, no. 6, September 2005 (2005-09), pages 615-623, XP008069352 ISSN: 1087-0571

## Description

The present invention relates to the preservation and storage of cells and in particular to cell based assays using cryogenically-preserved cells.

The application of high throughput screening (HTS) technologies has enabled a step change in drug discovery by providing the pharmaceutical industry with the opportunity to conduct cell based assays for the discovery and development of new therapeutic drugs. In the HTS process, drug candidates are screened for possible effects in biological systems and for the specificity of selected lead compounds towards particular targets. Primary screening has been addressed by the development of HTS assay processes and assay miniaturisation utilising the microtitre well plate format with 96, 384, 864, 1536 or greater miniaturised wells and are capable of allowing throughput levels of over 100,000 tests/day. This has established the need for large numbers of cells for each type of test, where for example, such tests are comprised of a specific genetically engineered cell. Lead compounds identified during the primary screening process are then required to undergo further refined screening and testing in a variety of assays in order to investigate the biological compatibility of the compound. Such assays may include receptor binding and enzyme activity assays, in addition to bioavailability, metabolism and toxicology.

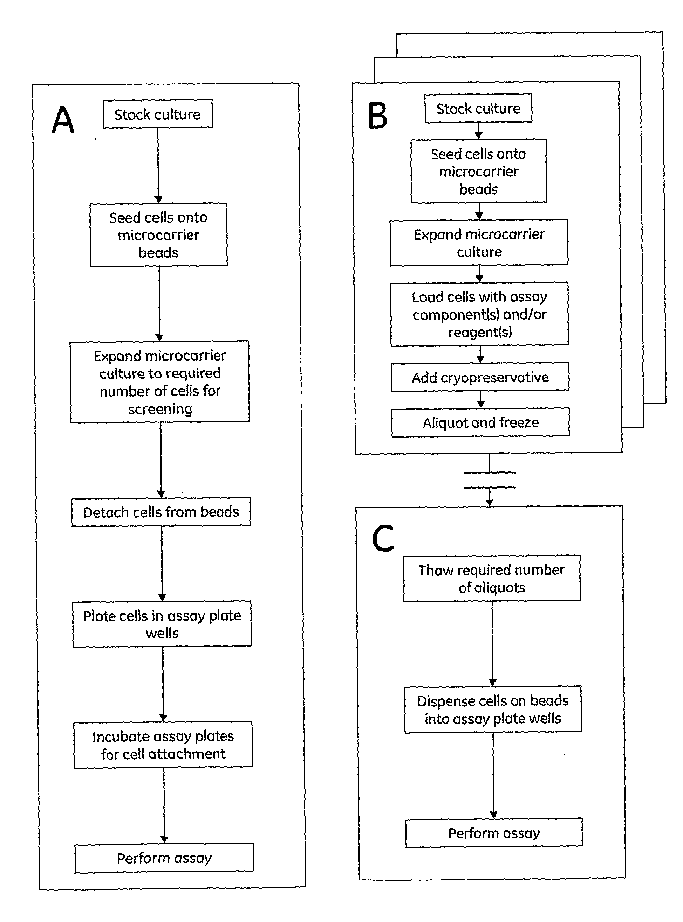

As a consequence, there is a significant challenge to overcome the "culturing" burden to allow multiple-billions of cells to be grown for each primary screen. Typically, cells are cultured in flasks and roller bottles. The growth of 1 x 10¹⁰ cells would enable 1,000,000 assays, based on 10,000 cells per assay. This would require approximately 2000 large flasks to be maintained, thereby consuming large amounts of resource, plastic-ware and growth media components. The use of Cytodex™ support particles or "microcarriers" (GE Healthcare) in cell culture methods has improved the yields of anchorage dependent cells by increasing the surface area for growth. The properties of these microcarriers include optimised size and density for maximum cell growth, a biologically inert matrix that provides a strong but non-rigid substrate for stirred cultures and transparency for easy microscopic examination of attached cells. Microcarriers can be used in either suspension cultures or monolayer cultures to increase the surface area of the culture vessels and perfusion chambers. The increased surface area enables the production of increased densities of cells, viruses and cell products. Cell adhering microcarriers incorporating scintillants have been used in cellular assays (US 2002/0094543, O'Beirne et al.). In a conventional process for growing and preparing cells for high-throughput drug screening (Figure 1A), a cell culture is initiated by seeding cells from a stock culture onto microcarrier beads. The microcarrier culture is then expanded in a continuous process until the culture produces the required number of cells to perform the drug screen. Once the required number of cells has been obtained as a single batch, cells are detached from the microcarrier beads by trypsinisation or by other means, the resulting cell suspension separated from beads and the cells plated out into wells of micro-well plates suitable for the intended assay. Plates are then incubated, typically overnight, to allow cells to attach to the surface of the wells and recover from the process of separation from the microcarrier beads prior to use in the screening assay.

The conventional process has two major of drawbacks in relation to production of cells and performing screening assays in a pharmaceutical drug screening environment. Firstly, the process may not be interrupted and once started, the cell culturing and screening process must be carried out from beginning to end in a continuous process. This can pose significant scheduling issues and may be vulnerable to equipment breakdown or operator availability. For example, if equipment to be used during the screening phase, e.g. a detection instrument, is inoperative, the cell culture will be wasted as it cannot be used. Conversely, if problems occur with the cell culture, for example, due to contamination, cells will not be delivered to fulfil the requirements of the screen and valuable detection instrumentation will sit idle. Secondly, in the conventional process, bulk cultured cells are typically recovered from the microcarrier support either by mechanical or by enzymatic means, prior to dispensing into microwell plates. In a continuous process, the subsequent incubation of cells in the assay wells is necessary to allow the cells to recover from metabolic shock and/or proteolytic damage incurred during the cell-bead separation, which is typically carried out using the protease, trypsin. This step requires the storage of large numbers of assay plates under tissue culture conditions, requiring significant equipment and personnel. The invention described herein provides a means for cell storage on beads and the subsequent utilisation of cryogenically stored cells in cellular assays. The invention provides rapid attainment of function for "assays on demand" and is expected to offer additional advantages associated with assay configuration.

According to a first aspect of the invention there is provided an assay method for measuring a cellular process comprising:
i) providing a population of cultured cells adhering to support particles in a liquid medium wherein said cells are pre-loaded after culture but before cryopreservation with at least one component for performing said assay and wherein said particles are adapted for cell attachment and/or cell growth;
ii) adding at least one cryo-preservative agent to the cultured cells;
iii) subjecting separate aliquots of said cells from step ii) to cryo-preservation at a reduced temperature;
iv) introducing one or more discrete samples of cells from at least one of said aliquots from step iii) into separate reaction vessels;
v) introducing into each said reaction vessel one or more further reagents for performing said assay;
wherein either said cells or at least one of said reagent or said further reagents comprises a detectable reporter that emits or is caused to emit an optical signal following combination of said reagents and said cells; and
wherein said optical signal is detected and/or quantitated as a means of measuring said cellular process.

In one embodiment, the assay component that is pre-loaded into cells may be a chemical reagent required to perform a cellular assay. Such chemical reagents include, but are not limited to, fluorescent, dye seniors, such as an ion sensitive dye or a fluorescent dye, enzyme substrates, enzyme co-factors, cell stimuli and receptor ligands. In this embodiment, one or more additional assay reagents may be combined with the cells in step v) of the method, so as to initiate the cellular process under investigation. Suitably, either at least one of the assay reagents added to the assay mixture, or alternatively the pre-loaded assay reagent, is labelled with a detectable reporter. In one example, the assay under investigation may be a cellular calcium release assay, in which case the pre-loaded assay reagent is a fluorescent calcium ion sensitive dye, such as Fluo-4. In another example, the cellular assay is based on detection of enzyme activity, such as activity from a reporter gene, in which case the chemical reagent pre-loaded into cells prior to cryo-preservation may be an enzyme substrate. Methods employing a variety of enzyme genes as a reporter gene in mammalian cells are well known (for a review see Naylor L.H., Biochemical Pharmacology, (1999), 58, 749-757). The reporter gene is chosen to allow the product of the gene to be measurable in the presence of other cellular proteins and is introduced into the cell under the control of a chosen regulatory sequence which is responsive to changes in gene expression in the host cell. Typical regulatory sequences include those responsive to hormones, second messengers and other cellular control and signalling factors. By selection of an appropriate regulatory sequence the reporter gene can be used to assay the effect of added agents on cellular processes involving the chosen regulatory sequence under study. One example of a reporter gene assay that may be performed according to the invention is a luciferase reporter gene assay, in which case the pre-loaded reagent is a luciferin substrate. Another example is a nitroreductase assay as described in WO 2005/118839 (GE Healthcare UK Limited).

Where the purpose of the cellular assay is to screen compounds for inhibitory activity against a cellular process measured in the assay, the pre-loaded assay reagent may be a stimulant for the cellular process, such that when cells are removed from cryo-preservation in order to perform the assay, the cellular process of interest is up-regulated ready for detection of inhibitory compounds. Where the purpose of the assay is to screen compounds for inhibition of ligand binding to a cellular receptor, the reagent may be a radiolabelled receptor ligand, such that when cells are removed from cryopreservation in order to perform the assay, the receptor of interest is already occupied with a detectable ligand, the displacement of which may be detected as an indicator of inhibitory activity in the course of the subsequent assay. It will be understood by those skilled in the art that in essence, any chemical reagent normally added to cells in order to perform a cellular assay may be pre-loaded into cells prior to cryo-preservation by the method of the present invention.

In a second embodiment, the cells are pre-loaded with a viral vector to transiently express a cell assay sensor or a detectable reporter. Such a reporter may be delivered to cells by means of a plasmid or a viral vector, typically an adenoviral vector. According to this embodiment, the reporter comprises a detectable protein, for example a fluorescent protein, including green fluorescent proteins and red fluorescent proteins (Tsien, R. et.al. Nat.Methods, (2005), 2(12), 905-9). Suitable fluorescent proteins include wild type GFP from *Aequorea Victoria* and derivatives of GFP such as functional GFP analogues in which the amino acid sequence of wild type GFP has been altered by amino acid deletion, addition, or substitution. Such fluorescent proteins include EGFP (Cormack, B.P. et al, Gene, (1996), 173, 33-38); EYFP and ECFP (US 6066476, Tsien, R. et al); F64L-GFP (US 6172188, Thastrup, O. et al); BFP, (US 6077707, Tsien, R. et al). Other examples of transiently expressed genetically encoded reporters include enzymes, such as luciferase, beta-lactamase and nitroreductase (as described hereinbefore), the expression of which may be measured by addition of a suitable enzyme substrate. In this embodiment, a vector comprising the DNA sequence encoding the reporter protein is added to cells prior to cryo-preservation such that when cells are recovered from cryo-preservation to perform the desired cellular assay, the cells are able to express the genetically encoded reporter required in the assay. It will be understood by those skilled in the art that in essence any genetically encoded reporter normally added to cells to produce transient expression of the reporter in order to perform a cellular assay may be pre-loaded into cells prior to cryo-preservation by the method of the present invention. agent is introduced to the assay mixture either prior to, or following step v); and wherein a change in said optical signal measured in the absence and in the presence of said test agent is indicative of the effect of said agent on said cellular process. Alternatively, the effect of a test agent may be determined by introducing a sample of the test agent either prior to or following step v) of the method and comparing the measurement of the optical signal with a control value which may be conveniently stored electronically in a database or other electronic format.

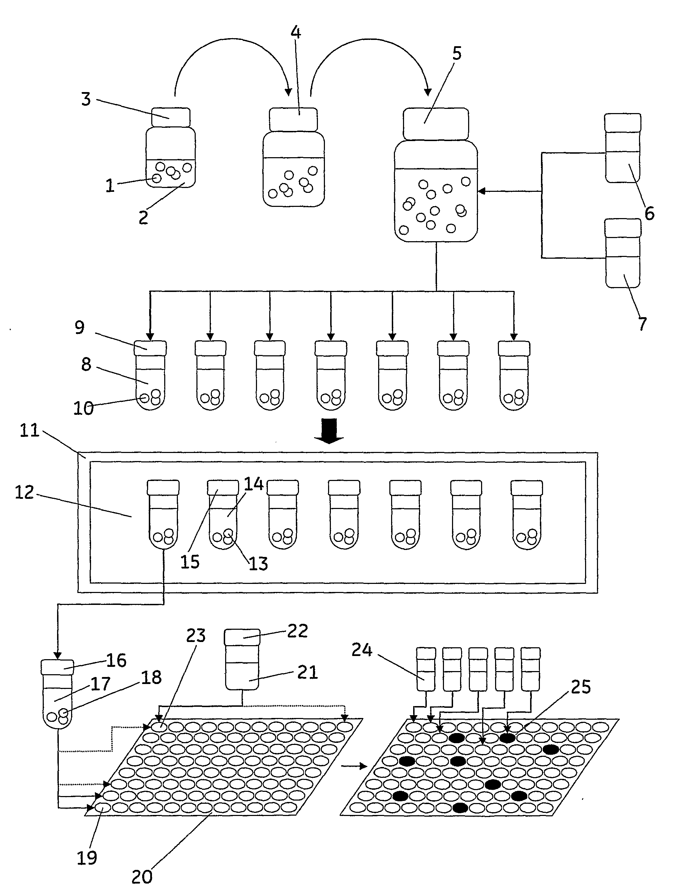

By cellular process, it is meant a normal process which living cells undergo and includes biosynthesis, uptake, transport, receptor binding, metabolism, fusion, biochemical response, growth and death. According to the method of the invention (with reference to Figure 2), cells are grown on a support [1] suspended in a liquid medium [2], whereby the support is modified or adapted as necessary to allow the adherence and growth of cells contained in a vessel [3]. Once cells have reached a chosen level of growth on the support, further cell growth may be achieved by transfer of cells on the support to a second vessel [4] of higher capacity, furnished with an additional quantity of the support material. Cell numbers may therefore be expanded in this manner, including if necessary further transfer of cells on the support to one or more vessels [5] of increasing size and capacity, until the desired number of cells is achieved. Cells growing on the support are then loaded with an assay component or reagent [6]. Cells growing on the surface are then prepared for cryo-preservation by the addition of a suitable cryo-preservative reagent [7] and the suspension of cells on the support [8] in cryo-preservative mixture [8] is separated into aliquots in vessels [9]. Vessels [15] are transferred to a low temperature environment [12] within a freezing chamber [11] to permit the cryo-preservation and long term storage of the cells on the support [13] suspended in the cryo-preservation medium [14].

For use of the cells in a cellular assay, one or more vessels [16] containing cryo-preserved cells adhering to the support suspended in liquid medium [17] are removed from low temperature storage and restored to ambient or elevated temperature to allow dispensing of cells on the support surface into the wells [19] of a multiwell plate [20] suitable for performing a cell based assay. Any additional assay reagents [21] in one or more containers [22] are added as required by the demands of the assay protocol to wells [23] containing cells on the support surface. Either the cells in the cell population, or at least one of the assay reagents is labelled with a detectable reporter that emits or is caused to emit an optical signal following combination of the reagents and cells and an optical signal is detected and/or quantitated as a means of measuring the cellular process. A test agent [24] may be added to assay wells and the assay allowed to proceed to generate the read-out of the assay to identify wells in which the test agent has activity in the assay [25].

Suitably, the support for use in the method of the invention is a particulate support and comprises any solid or semi-solid phase material suitable to enable the growth of attached cells. Suitably, the support is bathed in a cell growth medium and may be divided into portions by subdivision of a liquid suspension of the support. Suitable materials for the particulate support include but are not limited to plastic, glass or polymer particles or beads. Preferred particles include commercially available cell microcarriers, particularly cross-linked dextran microcarriers, for example Cytodex (GE Healthcare), contrary to earlier findings of Yanai, N and Masao T, (Biological Abstracts (1989) 88 (9), AB240) which reported that Cytodex was unsuitable for cryopreservation of adhered cells.

Cells suitable for use in the method of the invention may be a single population of cells of the same type, or may be a mixed population. Such cell types may be selected from adherent prokaryotic or eukaryotic cells, for example bacteria, yeast, insect cells or mammalian cells. Examples may include stem cells, differentiated stem cells, primary cells, transformed cells and genetically engineered cells, the cells characterised by a requirement for attachment to a particulate support surface for maintenance of cell viability and/or growth. The culture of cells on a support according to the present invention involves the use of standard cell culture techniques, e.g. cells are cultured in a sterile environment at 37°C in an incubator containing a humidified 95% air/5% CO₂ atmosphere. Alternatively, cells may be cultured in sealed vessels containing an atmosphere of air/5% CO₂. Vessels may contain stirred or stationary cultures. Various cell culture media may be used including media containing undefined biological fluids such as foetal calf serum, as well as media which is fully defined, such as 293 SFM II serum free media (Invitrogen, Paisley, UK). There are established protocols available for the culture of diverse cell types. (See for example, Freshney, R.I., Culture of Animal Cells: A Manual of Basic Technique, 2nd Edition, Alan R.Liss Inc. (1987); Freshney, R.I., Cloning ands Selection of Specific Cell Types in Culture of Animal Cells, 3rd Edition, Wiley-Liss Inc., (1994), p 161-178.)

In one embodiment, cells grown on the particulate support are engineered to stably express a cell assay sensor or detectable reporter, for example a reporter gene expressing a fluorescent protein, or expressing an enzyme, so as to provide a cellular assay readout.

In another embodiment, cells grown on the particulate support are engineered via addition of a plasmid or viral vector to the bulk culture prior to cryo-preservation to transiently express a cell assay sensor or detectable reporter that provides a cellular assay readout. In an alternative embodiment, cells grown on the particulate support may be engineered via addition of a plasmid or viral vector to an aliquot of cells recovered from cryo-preservation to transiently express a cell assay sensor or reporter that provides a cellular assay readout. Suitable vectors for transient expression include, but are not limited to, plasmids, adenovirus and baculovirus.

Reporter gene technology is widely used to monitor the cellular events associated with signal transduction and gene expression. In the context of the present invention, the term reporter gene is used to define a gene with a readily measurable phenotype that can be distinguished easily over a background of endogenous proteins. A reporter gene construct is comprised of an inducible transcriptional control element driving the expression of the reporter gene, for example using the Nuclear Factor of Activated T-Cells (NFAT) driving the expression of firefly luciferase (Zhong, H., Lee, D., Robeva, A., Minneman, K.P., Life Sci., (2001), 68(19-20), 2269-76). Suitable reporters include, but are not limited to, enzymes, fluorescent proteins, photo-proteins, fusion proteins and epitope tags. Examples of enzyme reporters include, but are not limited to, beta-galactosidase, alkaline phosphatase, beta-lactamase, luciferase and nitroreductase. Examples of fluorescent proteins include, but are not limited to, Aequorea victoria green fluorescent protein and variants thereof. Suitable photo-proteins include, but are not limited to, aequorin.

One example of a reporter gene assay that may be performed according to the present invention employs cells engineered by the introduction of a nucleic acid molecule comprising a nucleotide sequence encoding a fluorescent protein which may be derived from GFP or any functional analogue, such as EGFP, YFP, or BFP (Shaner, N.C., Steinbach, P.A., Tsien, R.Y., Nat. Methods, (2005), 2(12), 905-9), the nucleic acid molecule being operably linked to and under the control of an expression control sequence. Cells recovered from cryo-preservation and including engineered nucleic acid reporter gene sequences are cultured under conditions suitable for the expression of the protein of interest. The fluorescence emission of the GFP or functional GFP analogue is then monitored as a means of measuring the expression of the protein of interest. The method may be used to detect and compare the effect of a test agent on the expression of a protein in a cell. The term "test agent" in the context of the present invention, is intended to include agonist or antagonist stimulation by exogenously applied drugs, hormones, or alternatively through environmental stimulation by heat, shock, pH change, etc. By comparing the fluorescence emission obtained in the presence and also in the absence of the test agent, it is possible to determine the effect of the test agent on the expression and/or localisation of the protein(s) of interest in the cells.

Another example, of a reporter gene assay is based on the use of bacterial nitroreductase (NTR) and a cell-permeant cyanine dye analogue. Nitroreductase is an FMN-dependent enzyme isolated from Escherichia coli B. Expression of NTR has been demonstrated in a number of mammalian cells (US 5777190; Shah et al). The ability of this enzyme to reduce nitro groups of fluorescence-quenched nitro-group containing dyes has led to the development of a convenient gene reporter assay system based on the expression of NTR and a cell permeable quenched cyanine dye - CytoCy5S™ which acts as substrate for the enzyme, thereby permitting the use of NTR as a reporter of gene expression in living mammalian cells.

Methods to establish reporter gene assays are well known (Schenborn, E., Groskreutz, D., Reporter gene vectors and assays. Mol. Biotechnol., (1999), 13(1), 29-44). Suitably, the reporter gene is placed under the transcriptional control of a promoter or an enhancer with a minimal promoter. Alternatively, transient transduction of host cells may suitably be performed using adenoviral vectors (Hitt, M.M., Addison, C.L., Graham, F.L., Human adenovirus vectors for gene transfer into mammalian cells, Adv. Pharmacol., (1997), 40, 137-206.). The vector DNA molecule does not integrate into the host chromatin but exists as an extrachromosomal molecule with a lifetime of typically 24-96 hours, dependent on the cell type.

In a further embodiment, cells grown on the particulate support surface are engineered via addition of a plasmid or viral vector to the bulk culture prior to cryopreservation, or alternatively, to an aliquot of cells recovered from cryo-preservation to transiently express a cell assay component, for example a drug target protein.

In a further embodiment, cells may be pre-loaded with a chemical or biochemical assay reagent, either alone, or in combination with engineered components, while the cells are in bulk culture on the support, by addition of a solution of the reagent such that the cells are ready for use in the chosen assay after recovery from cryo-preservation without further manipulations or additions. Suitable methods for introduction of such assay reagents include the use of cell permeable dyes, e.g. a fluorescent calcium sensor such as Fluo-4 (Invitrogen, Paisley UK), delivery of molecules using peptide carriers, e.g. Chariot (Active Motif, Carlsbad, US), or loading of molecules in to cells by osmotic shock (Ahmad, F., Li, P.M. Meyerovitch, J., Goldstein, B.J., Osmotic loading of neutralizing antibodies demonstrates a role for protein-tyrosine phosphatase 1 B in negative regulation of the insulin action pathway. J. Biol. Chem., (1995), 270(35), 20503-8).

Alternatively, cells may be pre-loaded with a cellular assay component or reagent via covalent attachment of the reagent to the particulate surface prior to cell growth such that the reagent becomes incorporated into the membrane or cytoplasm of cells growing on the surface as the cells overgrow the surface. Preferably, covalent attachment of the reagent to the surface is implemented via a flexible linking and spacing moiety to allow uptake of the component into cells. Optionally the linking moiety may incorporate a cleavable linker acted on by a cellular component such as an enzyme, thereby allowing detachment of the component from the surface and incorporation into cells.

Examples of such chemical or biochemical assay reagents include an ion sensitive dye (e.g. Ca²⁺, K⁺), an enzyme substrate, an enzyme co-factor, a reporter gene substrate, a fluorescent dye, a pH sensitive dye, an energy transfer dye and a fluorescent sub-cellular imaging marker. Particular examples of enzyme substrates include luciferin, CytoCy5-S, coelenterazine. Examples of ion sensitive dyes include Fura-2 and Fluo-4.

Optionally, the cells may be additionally treated with a marker that permits visualisation and/or quantification of cells cultured on the surface of the support particles as an aid to enable assay standardisation. Cells growing on the surface of the support may be labelled with a detectable label to allow quantification of cells delivered to cellular assays, measurement of the detectable label being used to normalise the assay signals to account for variations in cell density on the support. Thus, cells grown on the particulate support may be loaded with a dye or other marker reagent by addition of the reagent to the bulk culture. Suitable markers include, but are not limited to, fluorescent DNA stains, for example Hoechst 33342 and DRAQ5 (BioStatus, UK) or membrane stains such as Dil and DiO (Invitrogen, Paisley, UK). Optionally, cells may be alternatively or additionally labelled with a detectable label that reports cell viability, for example, Calcein AM ester (Invitrogen, Paisley, UK). Optionally, cells may be alternatively or additionally labelled with a detectable label which to aids in image analysis segmentation in cellular assays, suitable markers include fluorescent DNA stains, for example DRAQ5 (BioStatus, UK).

In a further embodiment, cells grown on the particulate support surface may be subjected to growth arrest by addition of a cell cycle inhibitor, for example mitomycin C (Vasudevan, C., Fursov, N., Maunder, P., Cong, M., Federici, M., Haskins, J.R., Livelli, T., Zhong, Z., Improving high-content-screening assay performance by using division-arrested cells. Assay Drug Dev. Technol., (2005), 3(5), 515-23; Fursov, N. et al., "Improving Consistency of Cell-Based Assays by Using Division-Arrested Cells. Assay Drug Dev.Technol., (2005), 3,(1), 7-15), prior to cryo-preservation. Division arrest of cells permits cells growing on the support surface to be held in a maintenance state under standard tissue culture conditions without the number of cells becoming too high to be supported by the available growth area, providing further flexibility in the timing of use of the cells in an assay following recovery from cryo-preservation.

In one embodiment, the particulate support may contain a detectable marker for use as an internal assay standard for data normalisation, for example a fluorescent dye, a pH sensitive dye, or a solid state sensor. Use of such a detectable marker allows determination of the amount of the particulate support present in a cellular assay, measurement of the detectable label being used to normalise the assay signals to account for variations in the amount of particulate support (and hence variations in cell number) between assays. Alternatively, the detectable marker enables discrimination of two or more cell types grown separately on differentially encoded surfaces when such surfaces are combined together in the same cellular assay. Examples of different cell populations may include cells differing in phenotype or genotype, for example normal and tumour cells; cells differing in cell engineering, for example engineered and non-engineered cells; cells differing in treatment, for example pre-loading with different dyes, sensor or reporters.

A mixture of two or more populations of cells in an assay wherein the discrete populations of cells are carried on corresponding discrete populations of identifiable support particles enables advances in assay complexity over that achievable with particles in the absence of particle encoding. Examples of such assays include, multiplexing of assays and comparison of signals from responsive and non-responsive cells. Multiplexing may be used, for example, to achieve measurement of reporter gene activity from two response elements using the same reporter gene and substrate. In this approach, signals can be discretely assigned to one of two populations of cells wherein the two populations of cells are engineered with the same reporter gene under the control of the two different response elements and the two populations of cells are supported on two discrete and identifiable populations of particles. Similarly, measurement of assay signals from responsive and non-responsive cell types, for example where one cell type lacks a key component of a signalling pathway, can be achieved by provision of the cells for the assay on two distinguishable types of support particle. To achieve differentiation, each different population of cells may be distinguished one from the other by encoding the support material upon which the cells are growing such that recognition of the coding by suitable detection means in the course of the assay measurement enables an apportionment of the detected assay signal to discrete populations of cells present in the assay. Methods suitable for encoding the support include, but are not limited to, physical means such as size and shape, optical means such as dye colour or fluorescence, and electronic means such as inclusion of radio-frequency encoding.

In one embodiment, the particulate support may contain a detectable marker comprising part of the assay signal generation procedure, for example a fluorescent dye or a quenching dye. In such applications, the particulate support carries or incorporates one or more components which generate a detectable signal indicative of a biological event occurring in or on cells associated with the support during the course of the assay. Examples of such signal generation means associated with the support include, but are not limited to, incorporation of fluors for example a fluorescent dye comprising a FRET or BRET partner, environmentally sensitive sensors, or solid state sensors. In one embodiment, a fluorescent dye is incorporated into the support for the purposes of energy transfer to accomplish a shift in emission wavelength of a signal originating from cells in fluorescence assays or to detect loss of cells or a change in cell shape via a FRET interaction between a cellular fluor and the fluor of the support. FRET (Fluorescence Resonance Energy Transfer) is a distance-related process in which the electronic excited states of two dye molecules interact without emission of a photon. See, Forster, T., "Intermolecular Energy Transfer and Fluorescence", Ann. Physik., Vol.2, p.55, (1948). One result of this interaction is that excitation of a donor molecule results in fluorescence emission from an acceptor molecule. For FRET to occur, suitably the donor and acceptor dye molecules must be in close proximity (typically between 10-100 Å), since energy transfer efficiency decreases inversely as the 6^{th} power of the distance (r) between the donor and acceptor molecules. Suitably, in the present invention, either the cell or the support may carry a donor fluor or acceptor fluor and vice versa. By donor, it is meant that the dye moiety is capable of absorbing energy from light and emits light at wavelength frequencies which are at least partly within the absorption spectrum of the acceptor. By acceptor, it is meant that the dye moiety is capable of absorbing energy at a wavelength emitted by a donor dye moiety. Suitably, there is overlap between at least a portion of the emission spectrum of the donor dye molecule with the absorption spectrum of the acceptor dye molecule. Suitably, the wavelength of the emission maximum of the acceptor dye is longer that the wavelength of the emission maximum of the donor dye. In one format of a FRET pair, the acceptor molecule can be a non-fluorescent (or quenching) moiety, which is in close proximity to donor fluorophor. In this format, excitation of the donor dye results in energy transfer to the non-fluorescent acceptor with energy dissipation as heat rather than fluorescence. Separation of the donor and acceptor moieties, for example by cleavage of a connecting bond, removes the quenching effect of the acceptor and allows fluorescence emission from the donor on excitation.

These characteristics of FRET may be applied to the present invention to permit cellular assays to be carried out wherein the support particles on which cells are grown become a component part of the assay. Such assays include assays for detecting toxic or other effects of test compounds on cell morphology by labelling cells on particles each with one of a FRET donor and acceptor pair, wherein growth of cells firmly attached to the surface of the beads brings the donor and acceptor moieties in to sufficient proximity for FRET to occur. A change in shape of the cells, for example rounding of cells in response to a toxic compound, will increase the distance between the FRET partners and a consequent modulation of the observed signal.

In a still further embodiment of the invention, the particulate support comprises physical selection means wherein the support surface and associated cells are subjected to sorting by mechanical or fluidic means prior to or after cryo-preservation. In some applications of the method of the invention, it may be advantageous to perform isolation of particular cell populations prior to cryo-preservation, for example, to separate out and discard particles with non-viable cells or to separate out and retain particles with cells expressing a cellular marker introduced using a vector. In further applications of the method of the invention, it may be advantageous to perform isolation of particular cell populations after recovery from cryopreservation and following a cellular analysis to identify and isolate a desired phenotype of cell arising subsequent to a differentiation stimulus. This may be the case for example where the cells cultured on the support surface are a stem cell line and the cellular assay has been performed to screen a range of treatments or conditions that are suitable for differentiation of the stem cells to a desired phenotype. Here, it would be desirable to be able to sort and retain any support particles carrying the differentiated cell phenotype. To achieve such separation, the surface, the interior, or the bulk of the support particle may be modified to alter its physical properties, including for example, differential density, differential magnetic properties, or differential dissolution in defined aqueous solutions, so as to aid in selection and/or separation of discrete cell populations carried on individual support particles.

Measurements of the assay signal using fluorescence detection may be made using instruments incorporating photo-multiplier tubes as detectors, or alternatively by imaging using a charge coupled device (CCD), or a complementary metal oxide semiconductor (CMOS) imager. For example, when assays are performed in a microwell plate format, area imaging of all of the wells of a microwell plate may be conveniently performed using a LEADseeker™ imaging system (GE Healthcare), allowing imaging of high density microwell plates in a single pass. Imaging is quantitative and fast, and instrumentation suitable for imaging applications can now simultaneously image the whole of a multiwell plate. Where an assay is to be formatted for the determination of the activity of a test agent on a cellular parameter, the assay may be performed under continuous measurement allowing kinetic studies to be performed in real time. Alternatively, where analysis of individual cell responses is required, suitable high-throughput sub-cellular imaging instrumentation may be used, for example IN Cell Analyser 1000 (GE Healthcare).

The method of the present invention departs significantly from and offers a number of advantages over the prior art process described above and illustrated by reference to Figure 1A. According to the present invention, (Figure 1B and 1C) the cell culture (B) and screening assay (C) phases of the process are separated into two non-contiguous parts. In the cell culture phase (B) cells are grown in microcarrier culture to a required cell number which may be equal to or less than that required to fulfil the requirements of a screen. An assay component or reagent is pre-loaded into cells and a cryo-preservative reagent is added to the bulk culture and the culture subdivided into aliquots and the aliquots frozen and stored (essentially indefinitely) in cryogenic storage. This process may be repeated as required in sequence or in parallel to accumulate cryogenically stored aliquots of cells sufficient to meet anticipated screening demands. To perform a screening assay (C) one or more aliquots of frozen cells on microcarrier beads are thawed and the beads dispensed into wells of micro-well plates suitable for the intended assay and the cells used immediately in the assay.

Furthermore, the method of the present invention overcomes the drawbacks of the conventional process described above. Firstly, the processes of cell culture and screening are now temporally separated. The cell culture process may be carried out in temporal and/or geographical isolation from the screening process. Cells may be cultured and expanded in numbers in a single batch or multiple batches at a convenient place and/or time determined solely by the demands and requirements of the cell culture process without regard to the scheduling or equipment availability of the screening process. The resulting aliquots of frozen cells on microcarrier beads may be stored as a stock consumable ready to meet the requirements of screening at a later time and may be readily transported from the culture laboratory to a distant screening facility on demand. In addition to the benefit of de-coupling the cell growth process from the screening process, the method of the present invention avoids the requirement to culture cells in the assay wells prior to assay. Since the cells are recovered from cryogenic storage and aliquoted directly into assay wells and the assay performed with the cells still on the beads, the cells are not subjected to metabolic or proteolytic injury associated with removal from the culture surface. This allows screening to be started directly from a frozen stock of cells without the need to hold an inventory of cells growing in assay plates prior to screening. Since cells are supplied from frozen stock in a 'just-in-time' manner, only those cells required for the immediate requirements of screening, for example one day's worth of assays, need to be used at one time. Hence, if problems occur in the screening process, due for example to equipment breakdown, the screening run may be interrupted and re-started with a fresh supply of cells following equipment repair. In the conventional method of the prior art, any delay in the screening phase is likely to compromise the whole screen, representing a waste of many weeks work, since once the coupled cell growth and screening phases have been initiated the scheduling of the entire process cannot be altered to accommodate any delay in the screening process.

The invention is illustrated by reference to the following examples and figures in which:
Figure 1 illustrates by means of flow diagrams, a conventional assay procedure (1 A) and the assay procedure according to the present invention (1 Band 1 C).
Figure 2 is a schematic diagram illustrating the process according to the present invention.
Figure 3 is a plot showing a calcium release assay performed using cells grown on microcarrier beads and pre-loaded with Fluo-4 prior to cryopreservation.

### Example 1

### Assay of histamine induced calcium release in U20S cells

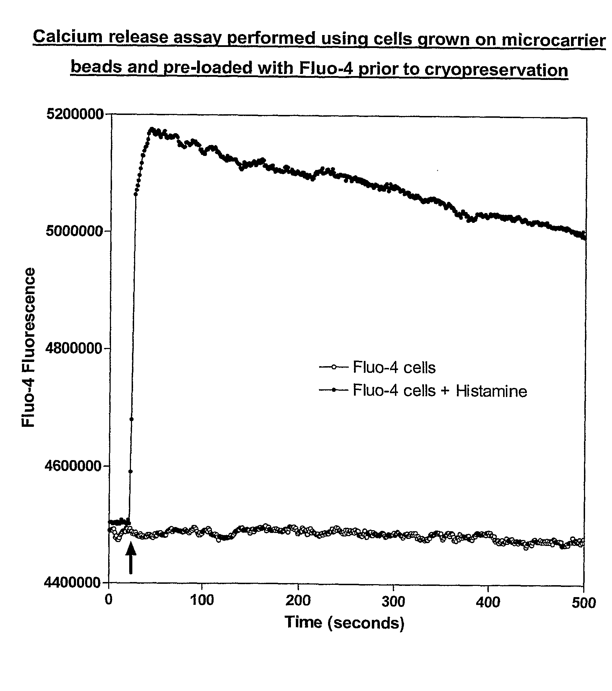

i) U2OS osteosarcoma cells were grown on collagen coated Cytodex 3 microcarriers (GE Healthcare) according to the Manufacturer's instructions in DMEM media (Invitrogen) supplemented with 10% (v/v) fetal bovine serum (Sigma). Once the culture had attained the required volume and cell density the cells on beads were loaded with 1µM Fluo-4 (Invitrogen). Microcarriers were allowed to settle, the growth media removed and replaced with calcium flux buffer (145mM sodium chloride, 5mM potassium chloride, 1mM magnesium sulphate, 10mM HEPES, 10mM D-glucose, 1mM calcium chloride containing 1% BSA, 1µM Fluo-4 (Invitrogen) and the cells incubated for 40 minutes at 37°C. Beads were allowed to settle, the calcium flux buffer removed and replaced with DMEM media supplemented with 10% (v/v) fetal bovine serum and glycerol (10% v/v final concentration) was added as a cryo-preservative. Fluo-4 loaded cells on beads were divided into aliquots of 8x10⁵ cells and the 1ml aliquots frozen to -140°C at a freezing rate of 1°C/minute.
ii) To set up the cell assay, vials of cryo-preserved cells were rapidly thawed in a water bath at 37°C and cells on bead suspensions placed in 1.5ml spectrofluorimeter cuvettes and equilibrated at 37°C for 30 minutes. Fluorescence measurements were acquired over time using a FluoroMax-3 spectrofluorimeter using excitation and emission settings for Fluo-4. Control cells showed a steady state fluorescence value of 4500000 fluorescence units (Figure 3, open circles). Addition of 250ml of a 40mM solution of histamine after 20 seconds (Arrow, Figure 3) to a second sample of cells on beads caused a rapid rise in Fluo-4 fluorescence to a value of 5170000 fluorescence units indicative of histamine stimulation of intracellular calcium release.

### Example 2

The following example illustrates how an assay of reporter gene activation may be performed.
i) U2OS osteosarcoma cells are grown on collagen coated Cytodex 3 microcarrier beads (GE Healthcare) according to the Manufacturer's instructions in DMEM media (Invitrogen) supplemented with 10% (v/v) fetal bovine serum (Sigma). Cells on beads are maintained in stirred vessels (Corning) on a controllable magnetic stirring platform (VarioMag) in a tissue culture incubator. Cultures are scaled up by trypsinisation of the microcarrier suspension to remove cells followed by addition of fresh microcarrier beads to supplement the total area available for cell growth and media to increase the volume of the culture. Once the culture has attained the required volume and cell density, the culture is mixed with an aliquot of adenovirus encoding a nitroreductase (NTR) reporter gene under the control of a Cyclic AMP (CRE) response element (GDS40001 Ad-A-Gene CRE-NTR, GE Healthcare) according to the supplier's instructions. Cells on beads are incubated with Ad-A-Gene CRE-NTR at a multiplicity of infection (moi) of 6 adenoviral particles/cell for 24 hours. Cells are prepared for cryo-preservation by addition of glycerol to a final concentration of 10% (v/v), and the microcarrier suspension is then divided into aliquots containing sufficient cells to seed 96 wells of a cell assay plate. Aliquots of microcarriers are frozen in a controlled rate freezer using a temperature drop of 1°C/minute until a temperature of - 70°C is achieved. Aliquots are then transferred to a storage freezer at -150°C.
ii) To perform the reporter gene assay an aliquot of cell/bead suspension is thawed in a water bath at 37°C and the suspension pipetted into the wells of a 96 well microtitre plate at 100ml/well. Reporter gene activation by test compounds is assayed by addition of compounds in 50ml of serum free media to wells. Forskolin at 10mmolar is used as positive control agonist. Cells are incubated for 10 min at 37°C and then 10ml of 10mmolar CytoCy5S NTR substrate (GE Healthcare) is added to each well. The cells are incubated for a further 12-16 h at 37°C, 5% CO₂, 95% humidity to allow activation of the NTR substrate. CytoCy5S fluorescence intensity (excitation: 647 nm; emission: 667 nm) is measured using a Tecan ULTRA microplate reader.

## Claims

1. An assay method for measuring a cellular process comprising:
i) providing a population of cultured cells adhering to support particles in a liquid medium wherein said cells are pre-loaded after culture but before cryopreservation with at least one component for performing said assay and wherein said particles are adapted for cell attachment and/or cell growth;
ii) adding at least one cryo-preservative agent to the cultured cells;
iii) subjecting separate aliquots of said cells from step ii) to cryo-preservation at a reduced temperature;
iv) introducing one or more discrete samples of cells from at least one of said aliquots from step iii) into separate reaction vessels;
v) introducing into each said reaction vessel one or more further reagents for performing said assay;
wherein either said cells or at least one of said reagent or said further reagents comprises a detectable reporter that emits or is caused to emit an optical signal following combination of said reagents and said cells; and
wherein said optical signal is detected and/or quantitated as a means of measuring said cellular process.

2. The method according to claim 1, wherein the assay component that is pre-loaded into cells is a chemical reagent required to perform a cellular assay.

3. The method according to claim 2, wherein said cells are pre-loaded with said reagent by addition of the reagent to the liquid medium prior to addition of said at least one cryo-preservative agent to the cells.

4. The method according to claim 2 or claim 3, wherein said reagent is selected from an ion sensitive dye, an enzyme substrate, an enzyme co-factor, a reporter gene substrate, a fluorescent dye, a cell stimulus, a receptor ligand and a fluorescent sub-cellular imaging marker.

5. The method according to claim 1, wherein said cells are pre-loaded with a plasmide or viral vector to transiently express a cell assay sensor or a detectable reporter.

6. The method according to claim 5, wherein said cells are engineered by addition of a plasmid or viral vector to said liquid medium prior to cryopreservation to transiently express a detectable reporter.

7. The method according to claim 5 or claim 6, wherein said reporter is an enzyme.

8. The method according to claim 5 or claim 6, wherein said reporter is a fluorescent protein.

9. The method according to any of claims 1 to 8, wherein said cells are selected from stem cells, differentiated stem cells, primary cells, transformed cells and genetically engineered cells.

10. The method according to claim 1, wherein said cells comprise a mixed population of cells.

11. The method according to any of claims 1 to 4, wherein cells are pre-loaded with said reagent through covalent attachment of the reagent to the particulate surface prior to cell growth such that the reagent becomes incorporated into the membrane or cytoplasm of said cells growing on the surface.

12. The method according to any of claims 1 to 11, wherein said cells are subjected to growth arrest by addition of a cell cycle inhibitor prior to cryopreservation.

13. The method according to any of claims 1 to 12, wherein said particulate support is encoded with a detectable marker.

14. The method according to claim 13, wherein said marker is selected from a fluorescent dye, a pH sensitive dye, a fluorescent protein or a solid state sensor.

15. A method according to any of claims 1 to 14, wherein the measurement of said cellular process is performed in real time.

## Patentansprüche

1. Assayverfahren zum Messen eines zellulären Prozesses, umfassend:
i) Bereitstellen einer Population kultivierter Zellen, die an Trägerteilchen in einem flüssigen Medium anhaften, wobei die Zellen nach der Kultur, jedoch vor der Kryokonservierung mit mindestens einer Komponente für die Durchführung des Assays vorbeladen sind und wobei die Teilchen für die Zellanheftung und/oder das Zellwachstum angepasst sind;
ii) Zugabe mindestens eines Kryokonservierungsmittels zu den kultivierten Zellen;
iii) Unterziehen getrennter Aliquots der Zellen aus Schritt ii) der Kryokonservierung bei einer reduzierten Temperatur;
iv) Einführen von einer oder mehreren diskreten Proben von Zellen von mindestens einem der Aliquote von Schritt iii) in getrennte Reaktionsgefäße;
v) Einführen in jedes Reaktionsgefäß eine oder mehrere weitere Reagenzien zur Durchführung des Assays;
wobei entweder die Zellen oder mindestens eines von dem Reagens oder den weiteren Reagenzien ein nachweisbares Reportermolekül umfasst, das nach erfolgter Kombination der Reagenzien und der Zellen ein optisches Signal emittiert oder veranlasst wird, es zu emittieren; und
wobei das optische Signal als ein Mittel zur Messung des zellulären Prozesses detektiert und/oder quantifiziert wird.

2. Verfahren nach Anspruch 1, wobei die Assay-Komponente, die in die Zellen vorgeladen wird, ein chemisches Reagenz ist, das erforderlich ist, um einen zellulären Assay durchzuführen.

3. Verfahren nach Anspruch 2, wobei die Zellen mit dem Reagens vorbeladen werden durch Zugabe des Reagens zu dem flüssigen Medium vor der Zugabe von dem mindestens einen Kryokonservierungsmittel zu den Zellen.

4. Verfahren nach Anspruch 2 oder Anspruch 3, wobei das Reagens ausgewählt ist aus einem ionenempfindlichen Farbstoff, einem Enzymsubstrat, einem Enzym-Cofaktor, einem Reportergen-Substrat, einem Fluoreszenzfarbstoff, einem Zellstimulus, einem Rezeptor-Liganden und einem fluoreszierenden subzellulären Bildgebungsmarker.

5. Verfahren nach Anspruch 1, wobei die Zellen mit einem Plasmid oder viralen Vektor vorbeladen sind, um transient einen Zellassay-Sensor oder ein nachweisbares Reportermolekül zu exprimieren.

6. Verfahren nach Anspruch 5, wobei die Zellen durch Zugabe von einem Plasmid oder einem viralen Vektor in das flüssige Medium vor der Kryokonservierung konstruiert werden, um transient ein nachweisbares Reportermolekül zu exprimieren.

7. Verfahren nach Anspruch 5 oder Anspruch 6, wobei das Reportermolekül ein Enzym ist.

8. Verfahren nach Anspruch 5 oder Anspruch 6, wobei das Reportermolekül ein fluoreszierendes Protein ist.

9. Verfahren nach einem der Ansprüche 1 bis 8, wobei die Zellen ausgewählt sind aus Stammzellen, differenzierten Stammzellen, Primärzellen, transformierten Zellen und gentechnisch veränderten Zellen.

10. Verfahren nach Anspruch 1, wobei die Zellen eine gemischte Population von Zellen umfassen.

11. Verfahren nach einem der Ansprüche 1 bis 4, wobei die Zellen mit dem Reagenz durch kovalente Bindung des Reagens an die Teilchenoberfläche vor dem Zellwachstum derart vorbeladen werden, dass das Reagens in die Membran oder das Zytoplasma der Zellen eingebaut wird, die auf der Oberfläche wachsen.

12. Verfahren nach einem der Ansprüche 1 bis 11, wobei die Zellen durch die Zugabe eines Zellzyklus-Inhibitors vor der Kryokonservierung einem Wachstumsarrest unterworfen werden.

13. Verfahren nach einem der Ansprüche 1 bis 12, wobei der partikuläre Träger mit einem detektierbaren Marker kodiert ist.

14. Verfahren nach Anspruch 13, wobei der Marker ausgewählt ist aus einem fluoreszierenden Farbstoff, einem pH-sensitiven Farbstoff, einem fluoreszierenden Protein oder einem Festkörpersensor.

15. Verfahren nach einem der Ansprüche 1 bis 14, wobei die Messung des zellulären Prozesses in Echtzeit durchgeführt wird.

## Revendications

1. Procédé de dosage pour mesurer un processus cellulaire comprenant les étapes consistant à :
i) fournir une population de cellules de culture adhérant à des particules de support dans un milieu liquide, dans lequel lesdites cellules sont pré-chargées après culture, mais avant cryoconservation avec au moins un composant pour effectuer ledit dosage et dans lequel lesdites particules sont adaptées pour fixer les cellules et/ou faire croître les cellules ;
ii) ajouter au moins un agent de cryoconservation aux cellules cultivées ;
iii) soumettre des fractions aliquotes séparées desdites cellules de l'étape ii) à une cryoconservation à température réduite ;
iv) introduire un ou plusieurs échantillons discrets de cellules provenant d'au moins l'une desdites fractions aliquotes de l'étape iii) dans des cuves réactionnelles séparées ;
v) introduire dans chaque dite cuve réactionnelle un ou plusieurs autres réactifs pour effectuer ledit dosage ;
dans lequel lesdites cellules ou au moins l'un dudit réactif ou desdits autres réactifs comprennent un rapporteur détectable qui émet ou est amené à émettre un signal optique après combinaison desdits réactifs et desdites cellules ; et
dans lequel ledit signal optique est détecté et/ou quantifié comme moyen de mesure dudit processus cellulaire.

2. Procédé selon la revendication 1, dans lequel ledit composant de dosage qui est pré-chargé dans les cellules est un réactif chimique nécessaire pour effectuer un dosage cellulaire.

3. Procédé selon la revendication 2, dans lequel lesdites cellules sont pré-chargées par ledit réactif par addition du réactif au milieu liquide avant addition dudit au moins un agent de cryoconservation aux cellules.

4. Procédé selon la revendication 2 ou la revendication 3, dans lequel ledit réactif est choisi parmi un colorant sensible aux ions, un substrat enzymatique, un cofacteur enzymatique, un substrat de gène rapporteur, un colorant fluorescent, un stimulus cellulaire, un ligand récepteur et un marqueur d'imagerie sous-cellulaire fluorescent.

5. Procédé selon la revendication 1, dans lequel lesdites cellules sont pré-chargées par un vecteur plasmidique ou viral pour exprimer de manière transitoire un capteur de dosage cellulaire ou un rapporteur détectable.

6. Procédé selon la revendication 5, dans lequel lesdites cellules sont élaborées par addition d'un vecteur plasmidique ou viral audit milieu liquide avant cryoconservation pour exprimer de manière transitoire un rapporteur détectable.

7. Procédé selon la revendication 5 ou la revendication 6, dans lequel ledit rapporteur est un enzyme.

8. Procédé selon la revendication 5 ou la revendication 6, dans lequel ledit rapporteur est une protéine fluorescente.

9. Procédé selon l'une quelconque des revendications 1 à 8, dans lequel lesdites cellules sont choisies parmi des cellules souches, des cellules souches différenciées, des cellules primaires, des cellules transformées et des cellules génétiquement élaborées.

10. Procédé selon la revendication 1, dans lequel lesdites cellules comprennent une population mixte de cellules.

11. Procédé selon l'une quelconque des revendications 1 à 4, dans lequel les cellules sont pré-chargées par ledit réactif via une fixation par covalence du réactif à la surface particulaire avant la croissance cellulaire de sorte que le réactif s'incorpore à la membrane ou au cytoplasme desdites cellules croissant sur la surface.

12. Procédé selon l'une quelconque des revendications 1 à 11, dans lequel lesdites cellules sont soumises à un arrêt de croissance par addition d'un inhibiteur de cycle cellulaire avant cryoconservation.

13. Procédé selon l'une quelconque des revendications 1 à 12, dans lequel ledit support particulaire est codé avec un marqueur détectable.

14. Procédé selon la revendication 13, dans lequel ledit marqueur est choisi parmi un colorant fluorescent, un colorant sensible au pH, une protéine fluorescente ou un capteur à l'état solide.

15. Procédé selon l'une quelconque des revendications 1 à 14, dans lequel la mesure dudit processeur cellulaire est effectuée en temps réel.
